# EUROPEAN PATENT SPECIFICATION

(11) **EP 4 223 326 B1**
(45) Date of publication and mention of the grant of the patent: **06.08.2025**
(21) Application number: 23154236.6
(22) Date of filing: 31.01.2023
(51) Int. Cl.: A61L 27/36

(54) **METHOD OF PREPARATION OF AMNIOTIC MEMBRANE-BASED MATERIAL**
VERFAHREN ZUR HERSTELLUNG VON MATERIAL AUF BASIS EINER AMNIONMEMBRAN
PROCÉDÉ DE PRÉPARATION D'UN MATÉRIAU À BASE DE MEMBRANE AMNIOTIQUE

(30) Priority: 02.02.2022 CZ 20220049
(43) Date of publication of application: 09.08.2023
(73) Proprietor: Narodni Centrum Tkani a Bunek a.s., 70800 Ostrava (CZ)
(72) Inventor: Sekorova, Sarka, 63800 Brno (CZ)
(74) Representative: Harber IP s.r.o.

(56) References cited:
- US-A1- 2004 181 240
- US-A1- 2011 189 301

## Description

### Field of art

The present invention relates to a method of preparing amniotic membrane-based material, to a lyophilization pad for use in this method, and to aminotic membrane-based materials obtainable by this method.

### Background art

Amniotic membranes are used for the production of materials for the reconstruction, protection and healing of injured tissues. Typically, these materials are produced by separating the amniotic membrane from the placenta, chorion, optionally removing the epithelial layer, placing the amniotic membrane on a carrier, and subsequently lyophilizing or freezing the tissue. The resulting product is therefore an amniotic membrane on a carrier. The carrier is typically a mesh textile carrier or a nitrocellulose carrier (e.g. CZ PV 2018-846).

The disadvantage of the nitrocellulose carrier is the fact that before application it is necessary to hydrate the amniotic membrane and the carrier, carefully remove the amniotic membrane from the carrier and then apply the amniotic membrane to the target spot. However, the hydrated amniotic membrane rolls up immediately after its separation from the carrier, and it is subsequently very difficult to determine its lateral orientation, which is crucial for the proper function of the graft, and it is difficult to manipulate the amniotic membrane.

Although the amniotic membrane hardly adheres to the mesh textile carrier and can be separated from the carrier easily, it often happens that it separates completely and prematurely and the surgeon then cannot determine its correct lateral orientation. Furthermore, it was shown on the histological sections that the amniotic membrane applied to the mesh textile carrier is not lyophilized in an adequate tension state. Within the histological structure of the amniotic membrane, condensation nuclei appear during freezing, thereby disrupting the natural structure of the amniotic membrane and causes a so-called tearing. This 3D structure is a key parameter for supporting the healing of defects and serves as a natural scaffold for re-epithelialization of the treated defect.

The present invention aims to eliminate the above problems.

US 2011/189301 A1 discloses a method for preparing a contact lens-shaped amniotic dressing, which comprises bringing an amniotic membrane in contact with a convex portion of a mold comprising a cylindrical portion and the convex portion at the upper part of the cylindrical portion such that the peripheral region of the membrane drapes around the cylindrical portion; placing a ring on the amniotic membrane covering the convex portion and fixing the ring on the side surface of the cylindrical portion; crosslinking the amniotic membrane; and cutting the amniotic membrane along the boundary between the convex portion and the cylindrical portion of the mold, to obtain a contact lens-shaped amniotic dressing.

### Disclosure of the invention

The present invention provides a method for processing an amniotic membrane into a material for reconstruction, protection and support of healing of damaged tissues, the method starting from placenta and comprising the following steps:
a) separation of the amniotic membrane from the chorion;
b) cleaning of the amniotic membrane in saline solution and mechanical cleaning of the amniotic membrane,
c) antibiotic lavage,
d) placing the amniotic membrane with the chorionic side onto a pad made of silicone, typically a medical grade silicone, the pad being provided with a tensioning system on its edges, and clamping, smoothing and tensioning (stretching) the amniotic membrane on the pad,
e) lyophilization of the material obtained in step d),
f) removing the amniotic membrane from the pad,
g) optionally fragmentation of the amniotic membrane into a circular shape,
h) inserting the lyophilized amniotic membrane into a sterile packaging.

The silicone pad is cylindrical. The circular shape of the base is the best shape for ensuring an even tension of the freeze-dried tissue in all directions (it follows the native sac-like shape of the amniotic membrane). The height of the cylinder is significantly smaller than the diameter of the base (e.g., at least 10 times smaller).

Silicone is a non-adhesive material that meets the requirements for easy work when applying and tensioning the amniotic membrane and also when removing the lyophilized tissue without damaging its structure.

The tensioning system comprises a recess along the side of the cylindrical pad, i.e. in the side wall of the pad; and a tensioning bar for insertion into the recess. The recess is present along the entire length of the circumference of the cylindrical pad. The tensioning bar is provided with fixing protrusions for better fixation and clamping of the amniotic membrane and for preventing shrinking and movement of the amniotic membrane on the pad during lyophilization. The tensioning bar also comprises fastening means, for example a tensioning screw.

The fixing protrusions of the tensioning bar can be, for example, protrusions fixed on one side of the tensioning bar, or moldings on the tensioning bar, or cutouts in the tensioning bar that are bent to one side away from the tensioning bar. Preferably, the fixing protrusions are configured to exert a force on the amniotic membrane directed from the center to the edge, for example they are pointed, with the tip directed towards the circular base on which the amniotic membrane is not placed (the bottom circular base).

Such tensioning system allows good smoothing and tensioning (stretching) of the membrane. Due to the circular shape and due to the recess and the tensioning bar, the amniotic membrane is stretched evenly, and the force applied in all areas of the membrane is adequate. Furthermore, displacement of the amniotic membrane during lyophilization is prevented. By tightening the fastening means, the tensioning/stretching of the amniotic membrane can be regulated to some extent. The tensioning system as described ensures adequate tensioning of the tissue to be lyophilized in all directions, and at the same time maintains a complete surface of the membrane available for an efficient course of lyophilization.

In a preferred embodiment, the silicone pad is provided with relief protruding pattern (embossing) on the surface of the circular base destined for placing the amniotic membrane (the upper base). The relief protruding pattern gets imprinted into the amniotic membrane surface which is adjacent to the pad, during the smoothing, tensioning and lyophilization of the amniotic membrane on the pad. This embossed and imprinted pattern will then remain visible on the resulting amniotic membrane-based material. For example, the chorionic side of the amniotic membrane can be marked (imprinted) in this way, so that it can be easily recognized by the surgeon during the application on the target spot, and incorrect application of the lyophilized membrane is prevented.

Preferably, the silicone cylindrical pad can also be provided with openings (vents) for moisture removal and for passage of air, especially on the circular base on which the membrane is placed (upper base) and/or on the cylinder side.

In the method of the invention, the step of separating the amnion from the chorion (step a)) is typically performed manually by blunt dissection, starting from the incision site of the amniotic sac and continuing toward the umbilical cord along the entire surface of the placenta. At the point where the amnion passes into the umbilical cord, this connection is severed by sectioning, e.g. using scissors or a scalpel.

The step of cleaning the amniotic membrane (step b)) in saline solution is typically performed by placing the amniotic membrane in saline solution. The amniotic membrane can be washed gently manually. Mechanical cleaning is typically performed by placing the amnion with the epithelial layer on the work surface and removing the residues from the surface of the tissue with a gentle movement of thumb and forefinger. The process of cleaning the amniotic membrane in saline solution and its mechanical cleaning can be repeated as needed, preferably 2x or 3x. At the end of this procedure, the amnion must be clean without blood clots or other residues and must be transparent. This can be verified, for example, by visual inspection.

Antibiotic lavage, i.e. cleaning of the amniotic membrane in a saline solution with the addition of an antibiotic, (in step c)) is preferably carried out in a bath containing a weight ratio of antibiotic to saline solution of 1:100 to 1:10, more preferably 1:60 to 1:40, most preferably 1:50. The antibiotic is preferably gentamicin. Preferably, the amniotic membrane is maintained in this bath for at least 25 minutes, more preferably at least 30 minutes.

Applying, smoothing and tensioning (stretching) the amniotic membrane (in step d)) is performed by placing the chorionic side of the amine membrane on the circular base of the silicone pad, manually smoothing the amniotic membrane over the surface of the circular base of the silicone pad, folding the amniotic membrane over the edge of the base and over the recess, and fixing the amniotic membrane by means of the tensioning bar inserted over the membrane into the recess. The tensioning bar is fixed by means of fastening means.

Lyophilization of the material (step e)) is carried out under normal conditions, i.e. under reduced pressure, e.g. 100 to 500 mTorr (13.3 to 67 Pa), and under reduced temperature, e.g. -20 to -65°C. After lyophilization, the material is packed in a sterile packaging, the sterile packaging is preferably sealed by welding.

It is usually convenient or necessary to fragment the amniotic membrane after lyophilization (optional step f)). This is done, like the entire procedure, aseptically. Preferably, the fragmentation is carried out in a laminar flow box. The most common fragment shapes are circular, but the membrane can be fragmented into other shapes as well. The size of the fragments is determined by the requirements for future use, so it can be quite variable.

Insertion into the sterile packaging (step g)) typically involves placing the material into a primary packaging, sealing the primary packaging, placing the primary packaging with the material into the secondary packaging, and sealing the secondary packaging.

Preferably, the placenta from one donor is always processed in one batch.

The present invention further provides an amniotic membrane-based material ("material") that is obtainable by the method according to the invention. This material consists of a lyophilized amniotic membrane with a substantially intact structure. The material may be preferably circular in shape. The material may have embossed relief pattern on one side of its surface.

The material of the present invention has been tested in practice. Within one year, about 250 corneal transplants consisting of the material of the invention were transplanted to patients. Unlike the previous amniotic membrane-based materials, the material of the present invention does not have to be attached to the cornea by stitches. The intervention thus can be only ambulatory and does not have to be performed at surgical operation room. This makes the transplantation more accessible to patients and less strenuous for the patient's organism, compared to transplantations using the materials known in the prior art.

### Brief description of drawings

Fig. 1 shows a histological section of a lyophilized amniotic membrane prepared by the method of the invention.
Fig. 2 shows a histological section of a comparative example of lyophilized amniotic membrane prepared by a process similar to the method of the invention, but with the lyophilization performed on a mesh textile carrier and without the membrane being kept in the stretched position by a tensioning system.
Fig. 3 to 5 show a pad. Fig. 3 is an axonometric view of a silicone cylinder with a recess, Fig. 4 shows an axonometric view of a silicone cylinder with a tensioning bar and fastening means (here a tensioning screw). Fig. 5 shows a detail of the inner side of the tensioning bar with fixing protrusions.
Fig. 6 is a view of the circular base of the cylindrical pad, provided with embossing.
Fig. 7 is a view of the circular base of the cylindrical pad, provided with embossing and holes for air passage and moisture removal.

### Examples of carrying out the invention

Before tissue collection at a collection facility, the necessary documentation and the mother's informed consent to tissue donation are completed, and a medical assessment of the mother's health to donate the placenta which contains the amniotic membrane is performed. The placenta is removed by trained personnel using aseptic technique during a delivery by Cesarean section. Immediately before the collection of placenta, samples of the mother's blood are taken for serological examination. The tissue is stored and then sealed in the primary Cover bag and in the secondary storage container. The thus collected and wrapped tissue is immediately stored in a refrigerator in the temperature range of 4 °C to 8 °C. Transport of the tissue to the tissue processing facility also takes place at refrigerator temperature.

In the tissue processing facility, an authorized employee checks the completeness and correctness of the data and the completeness of the documentation. After receiving negative serological test results, the tissue is released by the authorized employee for processing. Processing must be started no later than 24 hours after tissue collection.

The tissue from only one donor is processed in one batch, to avoid potential cross-contamination. Processing takes place in a laminar flow box, cleanliness classification of Grade A. After aseptic unpacking of the tissue in the laminar flow box, a control smear of the tissue for microbiological examination is performed. During processing, the worker visually evaluates the tissue - evaluates the color and structure of the tissue. If changes in color or changes in structure are found during processing, it is necessary to assess their appearance, extent and character. If defects are found on a smaller scale, the tissue is adjusted and the defects are removed. In the case of unsatisfactory tissue quality on a larger scale and extensive defects, the tissue is discarded after evaluation by the worker. Color changes that lead to disposal include a clearly visible green, yellow, or brownish-green color of the tissue.

### Separation of the amniotic membrane from the chorion (step a)

The incision site of the amniotic sac (around the entire circumference of the incision) is identified manually, at this point the separation of the amnion from the chorion is begun. These 2 membranes are gently separated from each other and the preparation is continued in the indicated place towards the umbilical cord over the entire surface of the placenta.

Dissection technique: blunt dissection.

After dissecting the amnion over the entire surface, the amnion remains fixed at the point of connection with the umbilical cord, this connection is severed by sectioning with scissors or a scalpel. The dissected amniotic membrane is placed in a container filled with a saline solution.

### Cleaning the amniotic membrane (step b)

The amnion is lightly and gently washed in a prepared container with saline solution. Next, the amnion is placed with its epithelial layer adjoining the working surface and the contact side of the amnion (with the chorionic membrane) is manually cleaned over its entire surface. During processing, the membrane is continuously hydrated with saline solution, and the non-fragmented parts of the tissue are placed into a clean container with saline solution to prevent tissue moisture loss. The cleaning procedure is repeated 2 to 3 times.

The finally cleaned amnion must be clean without blood clots or other residues, it must be transparent.

### Antibiotic lavage (step c)

A bath is prepared from a saline solution and gentamicin in a weight ratio of 50: 1. The membrane is then placed in the prepared bath and left in it for 30 minutes.

### Stretching the amniotic membrane on the lyophilization pad (step d)

The entire amnion is resected into several larger fragments (for example approx. 15x15 cm) using scissors or a scalpel. Each individual fragment is placed with the chorion side down on a lyophilization pad made of medical grade silicone. The amniotic membrane is then manually smoothed over the surface of the pad, the tissue is folded over the edges of the pad and fixed with a tensioning bar. The tensioning bar is provided with fixing protrusions on the inside. Before the final tightening of the tensioning bar, it is checked that the entire surface of the amniotic membrane is adhered to the lyophilization pad. If there are any bubbles under the amniotic membrane, they are gently pushed out (e.g. by a finger) to the edges of the pad. The adhesion of the tissue at the place of the embossed pattern is also checked. Subsequently, the tensioning bar is tightened using fastening means.

### Lyophilization (step e)

The amniotic membrane stretched on the lyophilization pads is placed into the lyophilizer. Lyophilization takes place according to the following lyophilization protocol: The initial temperature of the lyophilization cycle is 20 °C. By gradual freezing, the temperature gradually drops to -45°C. At this temperature, the tissue is maintained for 320 minutes. After the end of the first phase of the lyophilization cycle, primary drying takes place at a pressure of 400 mTorr (53 Pa) followed by drying at a pressure of 450 mTorr (60 Pa) and a gradual rise in temperature to 10°C. The lyophilization is then terminated.

### Fragmentation (step f)

After lyophilization, the lyophilization pads with the amniotic membrane are placed in a protective packaging and moved to a laminar flow box, cleanliness Grade A. In the laminar flow box, the amniotic membrane is removed from the lyophilization pad and fragmented with a trephine into circular grafts of various diameters, corresponding to the size of the selected trephine(s). The individual fragments are inserted into the primary packaging, which is welded with a pulse welder, and then inserted into the secondary packaging. The secondary packaging is also sealed with a weld. The entire process takes place under aseptic conditions. A representative sample of the amniotic membrane is sent to a certified laboratory to evaluate the sterility of the final product. Another representative sample is taken to evaluate the residual moisture of the final product, which must not be higher than 6%.

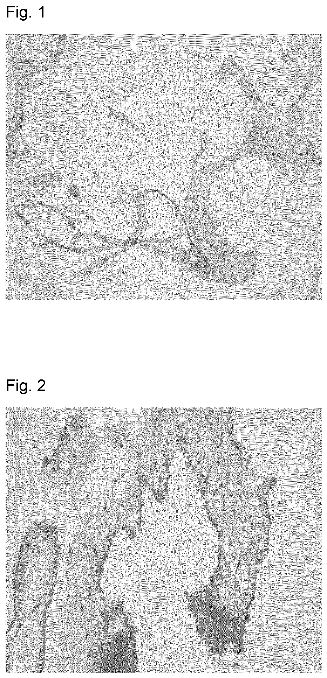

Fig. 1 shows a histological section of a lyophilized amniotic membrane prepared by the procedure described above, i.e. according to the invention. Fig. 2 shows a histological section of a comparative lyophilized amniotic membrane prepared by a similar procedure, differing in that instead of being lyophilized on a silicone pad provided with a tensioning system, the amniotic membrane was lyophilized on a mesh textile carrier; the section was made after removing the membrane from this carrier.

Histological sections were made at the Department of Pathology of the Faculty Hospital Brno, and are analyzed using light microscopy, magnification 200x, after staining with hematoxylin and eosin.

In Fig. 1, the well-preserved compact structure of the amniotic membrane is visible. On the other hand, in Fig. 2, significant tearing and pulling of the amniotic membrane structure is visible.

### Example of a lyophilization pad:

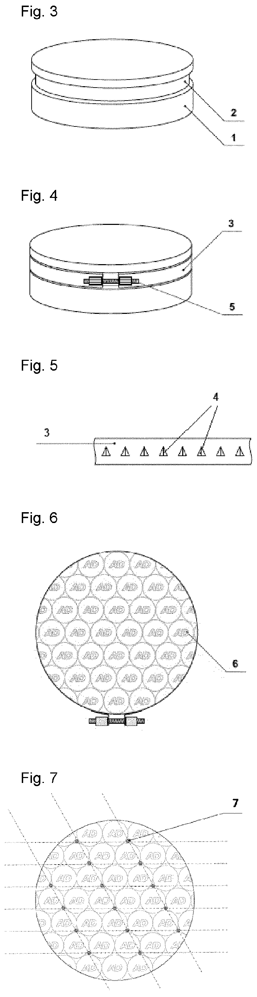

The lyophilization pad according to this example is shown in Fig. 3 to 5. The pad 1 is made of medical grade silicone, and has the shape of a cylinder with two circular bases and a side. The height of the cylinder is significantly smaller than the diameter of the base. A recess 2 is created in the shell around the entire circumference, the recess serves for inserting the tensioning bar 3. The tensioning bar is provided with fixing protrusions 4, which in this case are formed by cutouts in the bar, the cutouts being bent sideways (inwards when the bar is inserted into the recess of the pad). The tensioning bar is also provided with fastening means 5, in this example the fastening means is a tension screw.

The pad can be provided with embossing, i.e. relief protruding pattern 6, as shown in the embodiment of Fig. 6. Fig. 7 shows another possible embodiment of the pad, which is provided with holes 7 for moisture removal and air passage located on the circular base.

## Claims

1. Method for processing an amniotic membrane into a material for reconstruction, protection and healing of damaged tissues, the method starting from placenta and comprising the following steps:
a) separation of the amniotic membrane from the chorion;
b) cleaning of the amniotic membrane in saline solution and mechanical cleaning of the amniotic membrane,
c) antibiotic lavage,
d) placing the amniotic membrane with the chorionic side onto a cylindrical pad (1) made of silicone, the pad (1) being provided with a tensioning system on its edges, and clamping, smoothing and tensioning the amniotic membrane on the pad (1), wherein the tensioning system comprises a recess (2) along the side of the cylindrical pad (1); and a tensioning bar (3) for insertion into the recess (2), wherein the tensioning bar (3) is provided with fixing protrusions (4) for fixation and clamping of the amniotic membrane and for preventing shrinking and movement of the amniotic membrane on the pad during lyophilization, and the tensioning bar (3) further comprises fastening means (5),
e) lyophilization of the material obtained in step d),
f) removing the amniotic membrane from the pad (1),
g) optionally fragmentation of the amniotic membrane into a circular shape,
h) inserting the lyophilized amniotic membrane into a sterile packaging.

2. The method according to claim 1, wherein the silicone pad (1) is provided with relief protruding pattern (6) on the surface of the circular base onto which the amniotic membrane is to be placed.

3. The method according to any one of claims 1, wherein the silicone pad (1) is provided with openings (7) for moisture removal and for passage of air, preferably on the circular base onto which the amniotic membrane is to be placed and/or on the side of the cylindrical pad.

4. Cylindrical pad (1) for carrying out the method according to claim **1, characterized in that** it is made of silicone, the pad being provided with a tensioning system comprising a recess (2) along the side of the cylindrical pad (1), and a tensioning bar (3) for insertion into the recess (2), wherein the tensioning bar (3) is provided with fixing protrusions (4) for fixation and clamping of the amniotic membrane, and the tensioning bar (3) further comprises fastening means (5).

5. The pad according to claim 4, which is provided with relief protruding pattern (6) on the surface of the circular base onto which the amniotic membrane is to be placed.

6. The pad according to any one of claims 4 or 5, which is provided with openings (7) for moisture removal and for passage of air, preferably on the circular base onto which the amniotic membrane is to be placed and/or on the side of the cylindrical pad.

## Patentansprüche

1. Verfahren zur Verarbeitung einer Amnionmembran zu einem Material für die Rekonstruktion, den Schutz und die Heilung geschädigten Gewebes, ausgehend von der Plazenta, mit den folgenden Schritten:
a) Trennung der Amnionmembran vom Chorion;
b) Reinigung der Amnionmembran in Salzlösung und mechanische Reinigung der Amnionmembran;
c) Antibiotikaspülung;
d) Auflegen der Amnionmembran mit der Chorionseite auf ein zylindrisches Pad (1) aus Silikon, wobei das Pad (1) an seinen Rändern mit einem Spannsystem versehen ist, und Einspannen, Glätten und Spannen der Amnionmembran auf dem Pad (1), wobei das Spannsystem eine Aussparung (2) entlang der Seite des zylindrischen Pads (1) umfasst; und einen Spannstab (3) zum Einsetzen in die Aussparung (2), wobei der Spannstab (3) ist mit Fixiervorsprüngen (4) zur Fixierung und Klemmung der Amnionmembran sowie zur Verhinderung von Schrumpfung und Bewegung der Amnionmembran auf dem Pad während der Gefriertrocknung versehen, und wobei der Spannstab (3) weist außerdem Befestigungsmittel (5) auf,
e) Gefriertrocknung des in Schritt d) erhaltenen Materials,
f) Entnahme der Amnionmembran vom Pad (1),
g) optional Zerkleinerung der Amnionmembran in eine kreisförmige Form,
h) Einlegen der lyophilisierten Amnionmembran in eine sterile Verpackung.

2. Verfahren nach Anspruch 1, wobei das Silikonpad (1) auf der Oberfläche der kreisförmigen Unterlage, auf die die Amnionmembran gelegt werden soll, ein reliefartiges Muster (6) aufweist.

3. Verfahren nach Anspruch 1, wobei das Silikonpad (1) mit Öffnungen (7) zur Feuchtigkeitsableitung und zum Luftdurchtritt versehen ist, vorzugsweise an der kreisförmigen Unterlage, auf die die Amnionmembran aufgelegt werden soll, und/oder an der Seite des zylindrischen Pads.

4. Zylindrisches Pad (1) zur Durchführung des Verfahrens nach Anspruch 1, **dadurch gekennzeichnet, dass** es aus Silikon besteht und mit einem Spannsystem ausgestattet ist, das eine Aussparung (2) an der Seite des zylindrischen Pads (1) und einen Spannstab (3) zum Einführen in die Aussparung (2) umfasst, wobei der Spannstab (3) ist mit Fixiervorsprüngen (4) zur Fixierung und Klemmung der Amnionmembran versehen und wobei der Spannstab (3) ist mit Befestigungsmittel (5) versehen.

5. Pad nach Anspruch 4, das auf der Oberfläche der kreisförmigen Unterlage, auf die die Amnionmembran aufgelegt werden soll, ein reliefartiges Muster (6) aufweist.

6. Pad nach einem der Ansprüche 4 oder 5, das mit Öffnungen (7) zum Abführen von Feuchtigkeit und zum Durchlassen von Luft versehen ist, vorzugsweise an der kreisförmigen Unterlage, auf die die Amnionmembran aufgelegt werden soll, und/oder an der Seite des zylindrischen Pads.

## Revendications

1. Procédé de transformation d'une membrane amniotique en un matériau destiné à la reconstruction, à la protection et à la guérison des tissus endommagés, le procédé partant du placenta et comprenant les étapes suivantes :
a) séparation de la membrane amniotique du chorion;
b) nettoyage de la membrane amniotique dans une solution saline et nettoyage mécanique de la membrane amniotique;
c) lavage antibiotique;
d) placement de la membrane amniotique, côté chorion, sur un coussinet cylindrique (1) en silicone, le coussinet (1) étant muni d'un système de tension sur ses bords, et serrage, lissage et tension de la membrane amniotique sur le coussinet (1), le système de tension comprenant un évidement (2) le long du côté du coussinet cylindrique (1); et une barre de tension (3) destinée à être insérée dans l'évidement (2), où la barre de tension (3) est munie de protubérances de fixation (4) pour la fixation et le serrage de la membrane amniotique et pour empêcher son rétrécissement et son déplacement sur le coussinet pendant la lyophilisation, et où la barre de tension (3) comprend également des moyens de fixation (5);
e) lyophilisation du matériau obtenu à l'étape d);
f) retrait de la membrane amniotique du coussinet (1);
g) fragmentation éventuelle de la membrane amniotique en une forme circulaire;
h) insertion de la membrane amniotique lyophilisée dans un emballage stérile.

2. Procédé selon la revendication 1, où le coussinet (1) en silicone est muni d'un motif en relief (6) sur la surface de la base circulaire sur laquelle la membrane amniotique doit être placée.

3. Procédé selon l'une quelconque des revendications 1 à 2, où le coussinet (1) en silicone est pourvu d'ouvertures (7) pour l'évacuation de l'humidité et le passage de l'air, de préférence sur la base circulaire sur laquelle la membrane amniotique doit être placée et/ou sur le côté du coussinet cylindrique.

4. Coussinet (1) cylindrique pour la mise en œuvre du procédé selon la revendication 1, **caractérisé en ce qu'**il est en silicone, le coussinet étant pourvu d'un système de tension comprenant un évidement (2) le long du côté du coussinet (1) cylindrique et une barre de tension (3) à insérer dans l'évidement (2), où la barre de tension (3) est pourvue de protubérances de fixation (4) pour la fixation et le serrage de la membrane amniotique, et comprend en outre des moyens de fixation (5).

5. Coussinet selon la revendication 4, pourvu d'un motif en relief (6) sur la surface de la base circulaire sur laquelle la membrane amniotique doit être placée.

6. Coussinet selon l'une quelconque des revendications 4 ou 5, qui est pourvu d'ouvertures (7) pour l'évacuation de l'humidité et pour le passage de l'air, de préférence sur la base circulaire sur laquelle la membrane amniotique doit être placée et/ou sur le côté du coussinet cylindrique.
